# EUROPEAN PATENT APPLICATION

(11) **EP 4 707 375 A1**
(43) Date of publication of application: **11.03.2026**
(21) Application number: 24199290.8
(22) Date of filing: 09.09.2024
(51) Int. Cl.: C12M 1/00, C12M 1/26

(54) **SYSTEMS AND METHODS OF CULTURING CELLS BY PERFUSION**

(71) Applicant: Meatable B.V., 2333 BK Leiden (NL)
(72) Inventor: Quakkelaar, Caspar Marinus, 2333 BK Leiden (NL)
(74) Representative: Nederlandsch Octrooibureau

(57) **Abstract**

The present invention relates to systems and methods of culturing cells by perfusion. Such a system comprises a bioreactor system comprising a bioreactor for culturing cells by perfusion in a cell culture medium and at least one reservoir to controllably supply the bioreactor; a cell retention system connected to the bioreactor; a waste reservoir to receive waste from the cell retention system; and a feed system comprising a feed reservoir and a pump, the feed system connected to the at least one reservoir to controllably supply the at least one reservoir in the bioreactor system.

## Description

### Field of the invention

The present invention relates to systems and methods of culturing cells by perfusion.

### Background of the invention

According to the most recent United Nations estimations, the current world population is 7.9 billion in July 2022 [https://www.worldometers.info/es/poblacion-mundial/#ref-1] and it is expected to reach 10 billion around the year 2056. This increase will be heterogeneously distributed around the globe, with nine countries covering half the projected growth of the global population in the next 30 years, including India, Nigeria, Pakistan, Egypt, and the United States of America. Population and economic growth are major drivers of increased meat consumption. According to the United Nations Food and Agricultural Organization (FAO, https://www.oecd-ilibrary.org/agriculture-and-food/oecd-fao-agricultural-outlook-2022-2031_f1b0b29c-en), an estimated growth of 15% in global meat consumption is projected by 2031. On the other hand, the correlation between income growth and higher meat consumption is clearly demonstrable at lower income rates but once consumers reach an adequate standard of living, they become more sensitive to environmental, ethical, and animal welfare and health concerns.

For this reason, there is a growing interest in finding alternative protein sources which ideally will be sustainable and will contain the nutrients normally provided by meat in the human diet. Cultured meat arises as another alternative to traditional animal agriculture that aims to produce the skeletal muscle and adipose tissues that normally comprise animal meats, except using in vitro tissue and biological engineering techniques. Despite efforts to develop robust protocols for scalable generation of animal cell types from easily accessible and renewable sources, protocols for scaling up production often remains cumbersome, lengthy, and difficult to reproduce and/or has not been established yet. Thus there remains a need for culture methods that allow scalability of stem cells.

### Summary of the invention

In a first aspect, the invention relates to a system for continuously culturing cells (e.g., pluripotent cells) by perfusion using a bioreactor system comprising a bioreactor for culturing the cells by perfusion in a cell culture medium and at least one reservoir to controllably supply the bioreactor. The system further comprises a cell retention system (for example, an alternating tangential flow filtration system) connected to the bioreactor; a waste reservoir to receive waste from the cell retention system; and a feed system comprising a feed reservoir and a pump, with the feed system connected to the at least one reservoir to controllably supply the at least one reservoir in the bioreactor system.

The bioreactor system could be, for example, a modular bioreactor system, meaning the system could include a number of bioreactors (e.g., from 1 or 2 to 8 bioreactors, for example 1, 2, 3, 4, 5, 6 or 8), each connected to one or more reservoirs, and can be expandable or retractable to have a different number of bioreactors as needed. Additionally, a number of bioreactor systems (e.g. modular systems or single systems) could be running in parallel, and could, for example, be connected to the same feed system and/or waste system.

By providing a separate feed system with a feed reservoir and a pump, medium can be continuously refilled to the bioreactor system to ensure that medium levels in the bioreactor remain substantially constant for continuous cell culture even as waste is removed from the system to the waste reservoir. Thus, such a system allows for continuous cell culturing by perfusion without the need to pause for refilling the reservoir in the bioreactor as cells are cultured and waste is removed. This is especially useful as the system can culture cells continuously, for example, overnight or over the weekend due to the use of a feed reservoir with pump and separate waste reservoir. Additionally, because the feed and waste reservoirs are external to the bioreactor system, one or both can be much larger than the reservoir in the bioreactor system, making refilling and/or disposal of waste infrequent. Further by supplying medium from the feed reservoir to the reservoir in the bioreactor system and only then to the bioreactor, the pump and precision in supply of the feed system can be somewhat less than the accuracy needed for the supplying of the bioreactor. This makes the feed system more cost-effective as precision is not as critical.

In an embodiment, the bioreactor system further comprises a pump for the controllable supply from the at least one reservoir to the bioreactor. Such a pump can help to precisely supply the medium(s) from each of the at least one reservoir into the bioreactor for continuous cell culturing.

In an embodiment, the at least one reservoir in the bioreactor system is a plurality of reservoirs. Optionally, the at least one reservoir which controllably supplies the bioreactor is subject to temperature control, for example, refrigeration. Such temperature control of at least one reservoir can help to ensure ideal conditions for the medium and/or cell culturing.

In an embodiment, the feed system further comprises a temperature control system to control the temperature of the feed reservoir and any medium therein. Such a temperature control system could be, for example, a refrigeration system (e.g., a refrigerated biosafety cabinet) which houses the feed reservoir. Providing temperature control for the feed reservoir can ensure that the medium is stored in appropriate conditions until it is supplied to the at least one reservoir of the bioreactor system. This can also ensure a longer shelf-life of the medium.

In an embodiment, the feed reservoir holds more volume than the at least one reservoir, for example, 150% to 2000% more volume. Optionally, bioreactor is around from about 100ml - about 300 ml. By having a feed reservoir with a much higher volume, the feed reservoir can ensure that the bioreactor system is able to consistently supply the bioreactor with proper levels of medium for cell culture over longer periods of time. The feed reservoir can supply the at least one reservoir as needed, and only has to be refilled or replaced periodically, allowing the system to continuously culture cells without needing frequent manual intervention for refilling medium. Such a system can then continue to operate to culture cells, for example, overnight or over a weekend without requiring an attendant to be there (for refilling) despite the bioreactor system having reservoirs which would be depleted during such a time period. This is especially useful in smaller or experimental bioreactor systems, which have smaller bioreactors (e.g., from about 100 - about 300 ml in volume) and smaller reservoirs. The culturing can be performed over longer periods despite the smaller bioreactor system, thereby enabling longer culturing by perfusion and experimental results which can then be scaled. This can also be especially useful in modular systems, that can have from 1 or 2 to 8, for example 2, 3, 4 or more systems running in parallel.

In a further aspect, a method for continuously culturing pluripotent cells with perfusion comprises controllably supplying cell culture medium from a reservoir to a bioreactor in a bioreactor system; culturing the pluripotent cells with perfusion in a cell culture medium in the bioreactor; retaining the cultured cells using a cell retention system; removing waste from the system; and controllably supplying the reservoir from a feed system connected to the reservoir, the feed system comprising a feed reservoir and a pump. Such a method is able to continuously culture cells in a bioreactor system, removing the waste and precisely refilling the bioreactor as needed. The feed system is then able to continuously refill the one or more reservoirs in the bioreactor system such that the system can continue to culture cells by perfusion for long periods of time (e.g., 1-60 days, or as long as the cell and medium health would allow) without needing manual refilling or maintenance. Such a system then allows for more efficient cell culture with less manual work required, thereby decreasing the time and costs to culture cells through perfusion.

In an embodiment, the method operates such that the level of medium in the bioreactor stays within a set range. Optionally, the set range is within 10% of a set fill level, for example, if the set fill level is 75% of the bioreactor volume, the set range could be 65%-85%. Such a method can help to ensure that the bioreactor stays at a desired level for proper culturing of cells without risk of overflowing from overfilling.

In an embodiment, the supply from the reservoir to the bioreactor is controlled with a pump in the bioreactor system, and the supply from the feed system to the reservoir is controlled by the pump of the feed system. As the bioreactor needs very precise inputs to ensure proper culturing of cells, the pump and system for the supply to the bioreactor can be very precise, for example, a positive displacement pump and a mass flow controller. As the feed system only refills the bioreactor reservoir, the pump for the feed system does not need as much precision and therefore a more cost-effective pump can be used. Such a pump could be, for example, a peristaltic pump which could be pre-calibrated or other type of pump to controllably supply the reservoir in the bioreactor system.

In an embodiment, the method further comprises controlling a temperature of the medium in the feed reservoir and in the reservoir. This can be through a heat exchange system, for example, a refrigeration system, such as a refrigerated biosafety cabinet in which the feed reservoir is situated, or through another temperature control system.

Preferably, a cell used in the invention may be a "pluripotent stem cell". As used herein, the term "pluripotent stem cells" includes embryonic stem cells, embryo-derived stem cells, induced pluripotent stem cells and somatic cells, regardless of the method by which the pluripotent stem cells are derived. Accordingly, in certain embodiments the pluripotent stem cell is selected from the group consisting of embryonic stem cells, induced pluripotent stem cells, embryonic cell lines, somatic cell lines and and immortalized cell lines. In certain embodiments, the pluripotent stem cells are epiblast-derived stem cells (EpiSC). In certain embodiments, pluripotent stem cells express one or more markers selected from the group consisting of: OCT-4, Sox2, Klf4, c-MYC, Nanog, Lin28, alkaline phosphatase, SSEA-3, SSEA-4, TRA-1-60, and TRA-1-81. Exemplary pluripotent stem cells can be generated using, methods known in the art. "Induced pluripotent stem cells" (iPS cells or iPSC) can be produced by protein transduction of reprogramming factors in a somatic cell.

A pluripotent stem cell for use in the invention can be from any species. Embryonic stem cells have been successfully derived in, for example, mice, multiple species of non-human primates, and humans, and embryonic stem-like cells have been generated from numerous additional species. Thus, one of skill in the art can generate embryonic stem cells and embryo-derived stem cells from any species, including but not limited to, human, non-human primates, rodents (mice, rats), ungulates (cows, sheep, etc.), dogs (domestic and wild dogs), cats (domestic and wild cats such as lions, tigers, cheetahs), rabbits, hamsters, gerbils, squirrel, guinea pig, goats, elephants, panda (including giant panda), pigs, raccoon, horse, zebra, marine mammals (dolphin, whales, etc.) and the like.

Similarly, iPS cells can be from any species. These iPS cells have been successfully generated using mouse and human cells. Furthermore, iPS cells have been successfully generated using embryonic, fetal, newborn, and adult tissue. Accordingly, one can readily generate iPS cells using a donor cell from any species. Thus, one can generate iPS cells from any species, including but not limited to, human, non-human primates, rodents (mice, rats), ungulates (cows, sheep, etc.), dogs (domestic and wild dogs), cats (domestic and wild cats such as lions, tigers, cheetahs), rabbits, hamsters, goats, elephants, panda (including giant panda), pigs, raccoon, horse, zebra, marine mammals (dolphin, whales, etc.) and the like.

In certain embodiments, the pluripotent stem cell, or for use is an animal cell. In certain embodiments the pluripotent for use in the invention may be from an edible animal species.

In certain embodiments, the cell is from a mammal, preferably a non-human mammal. Preferably, the cell is an animal cell. In certain embodiments the cell is from an edible animal species.

Accordingly, in one embodiment, the cells produced by the method as described herein are suitable for human and non-human dietary consumption.

In certain embodiments, the pluripotent stem cells are of a livestock or poultry species.

Poultry species include but are not limited to domestic chicken, turkeys, ducks, geese and pigeons. In certain embodiments, the cells originate from common game species such as wild deer, gallinaceous fowl, waterfowl and hare.

Livestock species include but are not limited to domestic cattle, pigs, sheep, goats, lamb, camels, water buffalo and rabbits.

In certain embodiments, the cells are derived from an immortalized cell line. Exemplary cell lines include, but are not limited to, 3T3-L1 (mouse pre-adipocytes), buffalo rat liver cells (BRL 3A), chicken liver cells (LMH),

In certain embodiments, the cell is an adipocyte or a muscle cell originating from a domestic pig or domestic cattle.

In certain embodiments, the cell is an hepatocyte originating from a duck or a goose.

In certain embodiments, the cell is a pluripotent cell. Preferably the pluripotent cell is selected from the group consisting of embryonic stem cells, induced pluripotent stem cells, embryonic cell lines, and somatic cell lines. Preferably, when a pluripotent cell is cultured, the pluripotent stem cell is a porcine or a bovine pluripotent stem cell. Most preferably, a porcine pluripotent stem cell. In certain embodiments, the stem cell according to the invention is a porcine epiblast stem cell (pEpiSCs). Preferably a pluripotent stem cell according to the invention, or for use in the invention, is not a human cell.

In certain embodiments, the cells as used in the methods as described herein are described in patent application nos. WO 2024/084082, WO2024/170696 and WO2024/170702. Any cell described in any one of those patent applications may be useful in the systems and methods of the invention.

In an embodiment, the systems and methods are for the proliferation (i.e. expansion) of a pluripotent cell.

In an embodiment, the systems and methods as described herein can be used for differentiation of a pluripotent cell.

In certain embodiments, the culture medium is a basal medium that is supplemented with said medium components.

The term 'basal medium', as used herein, includes reference to a liquid medium that supports cellular growth by providing essential components for growth. A basal medium may be provided in liquid or powdered format. A basal medium that is not supplemented with any compound may enable cellular growth, but supplementation may be required for growth depending on the cell type. A basal medium may be supplemented with one or more components selected from the non-limiting group consisting of amino acids, lipids, sugars, carbohydrates, anions, cations, buffering agents, colorants, vitamins, antioxidants, hormones, enzymes, proteins and trace elements. In some embodiments, the basal medium as disclosed herein is a commercially available basal medium such as DMEM (Dulbecco's Modified Eagle Medium) and Ham's F- 12. In some embodiments, the basal medium is the medium described in any of patent application nos. WO 2024/084082, WO2024/170696 and WO2024/170702, which are incorporated by reference herein. Any of the media described in any one of those patent applications may be used as basal medium in the systems and methods of the invention.

The systems and methods off the invention may be used for the culture of a cell.

In certain embodiments, the methods as described in the various embodiments further comprise the step of:
- incorporating the cultured cell into a food product for animal, preferably human, consumption.

In a further aspect, the invention provides for a cell obtainable by or obtained by the methods as described in the various embodiments as described herein. In an embodiment, cells obtained by the method as described herein are suitable for human and non-human dietary consumption.

In yet a further aspect, the invention provides for a food product (also referred to as "foodstuff') comprising the cells produced, obtained by the methods and/or cultured in any of the mediums as described herein. In certain embodiments, the food product is for animal, preferably human, consumption.

A food product of the present invention may comprise one or more of minerals, synthetic substances, flavors, texture enhancers, nutritional additives, preservatives, and fats. In an aspect of the invention, the flavors are selected from one or more of essential oils, oleoresin (ESO), enzymes (ENZ), natural substances and extractives (NAT), non-nutritive sweetener (NNS), nutritive sweetener (NUTRS), herbs, spices, natural seasonings & flavorings (SP), and synthetic flavors (SY/FL), fumigant (FUM), artificial sweeteners and yeast extract. In another aspect of the present invention, the texture enhancers are selected from one or more of pureed plant material, guar gum, cellulose, hemicellulose, lignin, beta glucans, soy, wheat, maize and rice isolates and beet fiber, pea fiber, bamboo fiber, plant derived fiber, plant derived gluten, carrageenan, xanthan gum, lecithin, pectin, agar, alginate, natural polysaccharides, grain husk, calcium citrate, calcium phosphates, calcium sulfate, magnesium sulfate and salts. In another aspect of the present invention, the nutritional additives are selected from one or more of trace elements, bioactive compounds, endogenous antioxidants, A, B-complex, C, D, E vitamins, zinc, thiamin, riboflavin, selenium, iron, niacin, potassium, phosphorus, omega-3, omega-6, fatty acids, magnesium, protein, amino acids salt, creatine, taurine, carnitine, carnosine, ubiquinone, glutathione, choline, glutathione, lipoic acid, spermine, anserine, linoleic acid, pantothenic acid, cholesterol, Retinol, folic acid, dietary fiber and amino acids. In yet another aspect of the present invention, the fats are selected from one or more of saturated, monounsaturated, polyunsaturated fats, corn oil, canola oil, sunflower oil, safflower oil, olive oil, peanut oil, soybean, flax seed oil, sesame oil, canola oil, avocado oil, seed oils, nut oils, safflower and sunflower oils, palm oil, coconut oil, omega-3, fish oil, lard, butter, processed animal fat, adipose tissue, cellular agriculture derived fat essential oil and oleoresin. In another aspect of the present invention, the preservative and/or antioxidant is selected from one or more of: sodium salt, chloride salt, iodine salt. Nitrates, nitrosamines. butylated hydroxyanisole (BHA), butylated hydroxytoluene (BHT), sodium benzoate, potassium benzoate and benzene ascorbic acid, citric acid, potassium, monosodium glutamate (MSG), sulphur dioxide, sulphites, antibiotics. It is noted here that any one additive, flavor, texture enhancer, nutrient additive, fat/oil and/or preservative/antioxidant may supply more than one attribute to food product of the present invention.

In certain embodiments the food product of the present invention further comprises (cultured) adipocytes or myocytes. In certain embodiments, the food products comprises the cultured adipocytes or myocytes as described in patent application nos. WO 2024/084082, WO2024/170696 and WO2024/170702. Any cell described in any one of those patent applications may be useful in a system or method of the invention for culturing cells.

In yet a further aspect, the invention provides for a cultured fat product for animal, preferably human, consumption, comprising at least one cell obtainable by use of a system or method as described herein.

In yet a further aspect, the invention provides for a use of the cell obtained or obtainable by use of the system or the method as described herein for tissue engineering, optionally for the production of cultured meat.

### Brief description of the drawings

The present invention will be discussed in more detail below, with reference to the attached drawings, in which:
Fig. 1 depicts schematic view of a system for culturing cells; and
Fig. 2 depicts a schematic view of a continuous process for culturing cells.

### Description

### Definitions

Unless defined otherwise, technical and scientific terms used herein have the same meaning as commonly understood by one of ordinary skill in the art to which this disclosure belongs. One skilled in the art will recognize many methods and materials similar or equivalent to those described herein, which could be used in the practice of the present invention. Indeed, the present invention is in no way limited to the method.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the elements is present, unless the context clearly requires that there be one and only one of the elements. The indefinite article "a" or "an" thus usually means "at least one".

As used herein, the term "and/or" indicates that one or more of the stated cases may occur, alone or in combination with at least one of the stated cases, up to with all of the stated cases.

As used herein, with "At least" a particular value means that particular value or more. For example, "at least 2" is understood to be the same as "2 or more" i.e., 2, 3, 4, 5, 6, 7, 8, 9, 10, 11, 12, 13, 14, 15, ... ,etc.

The word "about" or "approximately" when used in association with a numerical value (e.g. about 10) preferably means that the value may be the given value (of 10) more or less 0.1% of the value.

"Expansion" refers to proliferation of cells with or without differentiation and may include no passaging, one passage or more than one passage and/or serial passages.

As used herein, a "medium" or "cell culture medium" refers to an aqueous based solution that provides for the growth, viability, or storage of cells. A medium as contemplated herein can be supplemented with one or more nutrients to promote the desired cellular activity, such as cell viability, growth, proliferation, differentiation of the cells cultured in the medium. A medium, as used herein, includes a serum replacement, a medium supplement, a complete medium or a cryopreservation medium. The pH of a culture medium should be suitable to the cells that will be grown. For example, most bacteria grow in pH 6.5-7.0, while most animal cell grow at a pH of from about 6.9 to about 7.6, for example at a pH of from about 7.2 to about 7 .4.

All patent and literature references cited in the present specification are hereby incorporated by reference in their entirety.

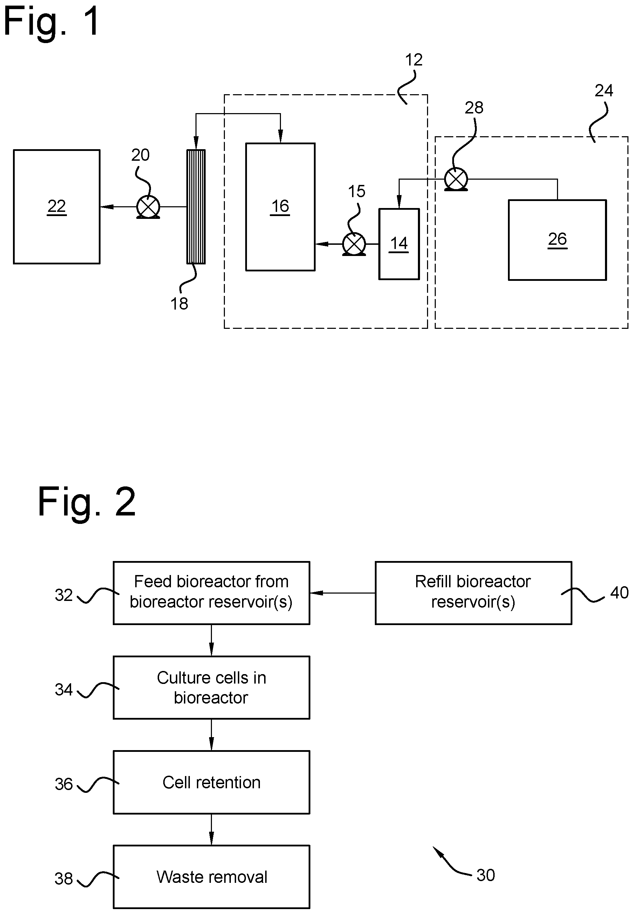

Fig. 1 depicts schematic view of a system for culturing cells 10, which includes bioreactor system 12 (with reservoir 14, pump 15 and bioreactor 16); cell retention system 18; pump 20, waste reservoir 22; and feed system 24 (with feed reservoir 26 and pump 28).

Bioreactor system 12 can be any bioreactor system, such as a modular bioreactor system for experimental purposes, such as the Ambr^{®}250 Modular system made by Sartorius, Headquartered in Göttingen, Germany. Such a system can include one or more reservoirs 14. While only one shown in Fig. 1, more could be included to provide different media to bioreactor 16. Reservoir 14 is fluidly connected to bioreactor 16 such that pump 15 and controller (not shown) can control for precise amounts of medium from reservoir 14 to flow into bioreactor 16. Such a precise pump 15 can be, for example, a positive displacement pump and a mass flow controller or any other suitably precise pump with controller. In some embodiments, reservoir 14 can have a temperature control system, for example, a refrigeration system or other type of temperature control system or apparatus to ensure a particular temperature of reservoir 14 and/or the medium within.

Bioreactor system 12 is connected to a cell retention system 18, for example, an alternating tangential flow (hereinafter "ATF") system, which receives medium from bioreactor 16. ATF system 18 is connected to waste reservoir 22, and pump 20 helps waste (e.g., spent medium) to flow from ATF system 18 to waste reservoir 22.

Feed system 24, particularly feed reservoir 26 is fluidly connected to reservoir 14 (of bioreactor system 12) such that pump 28 can cause medium from inside feed reservoir 26 to controllably flow to reservoir 14 in bioreactor system 12. Feed system 24 can include a temperature control system, for example, a refrigerated biosafety cabinet or a heating system in which feed reservoir 26 is stored to maintain a certain temperature of feed reservoir 26 and the medium within. Feed reservoir 26 can have a high volume, for example, 2 L - 5 L or 150% to 1000% or more of the volume of the reservoir 14 which feed reservoir 26 is supplying in the bioreactor system 12.

All connections between and within feed system 24, bioreactor system 12, cell retention system 18 and waste reservoir 22 can be through flexible tubing with tight sterile connections between the various reservoirs/systems/bioreactor.

Figure 2 depicts a schematic method 30 of continuously culturing cells through perfusion. During the culturing of cells by perfusion, media is fed from reservoir(s) 14 in bioreactor system 12 to bioreactor 16 (Step 32). This can be facilitated through one or more positive displacement pumps 15 and a mass flow controller to ensure precise feeding of the medium with components needed for the culturing of cells.

As used herein "perfusion" refers to the process of keeping culture cells alive by continuously feeding the cells with fresh media and removing spent media while keeping cells in culture. Perfusion is typically a continuous process. Perfusion culturing includes, but is not limited to continuous flow and semi-continuous flow, for example step-wise flow or staggered flow.

In step 34 the cells are cultured in the bioreactor, expanding, such as proliferating and/or differentiating, and step 36 then flows the cells and medium to the ATF system and back to the bioreactor 16 for cell retention. A diaphragm pump can be used to alternate the flow direction over a membrane surface where the cells are retained, and the waste can flow through and on to waste receptacle 22 (step 38). Due to the alternating flow, cells and media may be continuously moved from the bioreactor 16 into the ATF system 18 and back to the bioreactor 16, While an ATF system is shown and described, alternative cell retention systems could be used.

In order to continuously culture cells by perfusion, medium must be controllably but continuously fed from the reservoir(s) 14 within the bioreactor system 12 to the bioreactor 16 (step 32). In addition, waste (spent medium) must be continuously removed (step 38) to make space for new medium. As waste reservoir 22 is typically an external system connected to the bioreactor system 12 (via the ATF system 18), it is typically not limited in size. However, reservoir(s) 14 are part of the bioreactor system 12 and therefore are limited to a certain size, for example, 50-150mL. Thus, when culturing cells through perfusion in past systems, the culturing had to be stopped frequently to refill reservoir(s) 14. This resulted in either not being able to continuously culture cells (e.g., on nights or weekends when there were no lab technicians to refill reservoir(s) 14), or many manual tasks to continuously culture, needing a technician available every few hours to refill reservoir(s) 14.

In the methods and systems provided herein, continuous culturing by perfusion can be done in bioreactor system 12 through step 40, refilling reservoir(s) 14 of bioreactor system 12 by feed system 24, allowing for reservoir(s) 14 to continuously provided the medium needed for culturing as waste is removed to waste reservoir 22. This can be through a fluid connection between feed reservoir 26 and reservoir(s) 14, with pump 28 directing medium from feed reservoir 26 to reservoir(s) 14. As feed system 24 is external to bioreactor system 12, the size is not limited and feed reservoir 24 can have a much higher volume or capacity, for example 1L - 2L or more, or 150% to 1000% of the volume of reservoir(s) 14. Additionally, pump 28 and any temperature control systems are also not limited in size. Further, as medium is flowed only to reservoir(s) 14 and not directly into bioreactor 16, the precision of flow is not required to be as high, allowing for more economical components and easier access for refilling and maintenance.

The rate at which medium from feed system 24 is fed to reservoir 14 can be pre-set (and changed periodically) or could be automatically controlled, for example, based on data from sensors monitoring the cell growth and/or bioreactor medium levels. In some embodiments, the flow rate from feed reservoir to reservoir 14 could be set according to the expected rate at which medium will be consumed according to the stage of culture, and could be periodically changed, for example, changed daily as the cells grow and require more media for continued growth.

Thus, system 10 and method 30 provide a cost-effective way to continuously culture cells through perfusion. By providing and connecting cell retention, waste and medium refill systems to a bioreactor system, system 10 is able to continuously culture cells without the frequent need for operator oversight to refill medium used in the culturing process in order to maintain a stable volume in the bioreactor for cell culturing as waste is removed. Additionally, larger reservoirs, pumps and/or temperature control systems can be used in the process to further ensure continuous culturing operations. Such methods and systems can be especially useful when culturing with smaller (e.g., experimental) sized bioreactor systems which are very limited in reservoir size within the bioreactor system.

While the invention has been described with reference to exemplary embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiments disclosed, but that the invention will include all embodiments falling within the scope of the appended claims.

## Claims

1. A system (10) comprising:
a bioreactor system (12) comprising a bioreactor (16) for culturing pluripotent cells by perfusion in a cell culture medium, and at least one reservoir (14) to controllably supply the bioreactor (16);
a cell retention system (18) connected to the bioreactor (16);
a waste reservoir (22) to receive waste from the cell retention system (18); and
a feed system (24) comprising a feed reservoir (26) and a pump (28), the feed system connected to the at least one reservoir (14) to controllably supply the at least one reservoir (14) in the bioreactor system (12).

2. The system of claim 1, wherein the bioreactor system (12) further comprises a pump (15) for the controllable supply from the at least one reservoir (14) to the bioreactor (16).

3. The system of claim 1, wherein the at least one reservoir (14) in the bioreactor system (12) is a plurality of reservoirs.

4. The system of claim 3, wherein the at least one reservoir (14) which controllably supplies the bioreactor(16) is subject to temperature control.

5. The system of any of the preceding claims, wherein the feed system (24) further comprises a temperature control system to control the temperature of the feed reservoir (26) and any medium therein.

6. The system of claim 5, wherein the temperature control system is a refrigeration system which houses the feed reservoir (26).

7. The system of any of the preceding claims, wherein the feed reservoir (26) and the at least one reservoir (14) in the bioreactor system (12) are fluidly connected with flexible tubing.

8. The system of any of the preceding claims, wherein the feed reservoir (26) holds about 150% to 2000% more volume than the at least one reservoir (14).

9. The system of any of the preceding claims, wherein the cell retention system (18) is an alternating tangential flow system.

10. The system of any of the preceding claims, wherein the bioreactor is around 100ml - 300 ml.

11. A method for continuously culturing pluripotent cells with perfusion, the method comprising:
controllably supplying cell culture medium from a reservoir (14) to a bioreactor (16) in a bioreactor system (12);
culturing the pluripotent cells with perfusion in a cell culture medium in the bioreactor (16);
retaining the cultured cells using a cell retention system (18);
removing waste from the system; and
controllably supplying the reservoir (14) from a feed system (24) connected to the reservoir (14), the feed system (24) comprising a feed reservoir (26) and a pump (28).

12. The method of claim 11, wherein the method operates such that the level of medium in the bioreactor (16) stays within a set range.

13. The method of claim 12, wherein the range is with 10% of a set fill level, preferably wherein the set fill level is about 75%-80% of the bioreactor volume.

14. The method of any of claims 11-13, wherein the supply from the reservoir (14) to the bioreactor (16) is controlled with a pump (15) in the bioreactor system (12), and the supply from the feed system (24) to the reservoir (14) is controlled by the pump (28) of the feed system (24).

15. The method of any of claims 11-14, and further comprising controlling a temperature of the medium in the feed reservoir (26) and/or in the reservoir (14).
